(19) Europäisches Patentamt
European Patent Office
Office européen des brevets

(11) EP 1 567 121 B1

(12) EUROPÄISCHE PATENTSCHRIFT

(45) Veröffentlichungstag und Bekanntmachung des Hinweises auf die Patenterteilung:
**26.03.2008 Patentblatt 2008/13**

(21) Anmeldenummer: **03780059.6**

(22) Anmeldetag: **26.11.2003**

(51) Int Cl.:
***A61K 8/27*** *(2006.01)* ***A61K 8/36*** *(2006.01)*
***A61K 8/86*** *(2006.01)* ***A61K 8/39*** *(2006.01)*
***A61Q 17/04*** *(2006.01)* ***A61K 33/30*** *(2006.01)*
***C01G 9/02*** *(2006.01)* ***C08K 3/22*** *(2006.01)*
***C08K 9/04*** *(2006.01)* ***C09C 1/04*** *(2006.01)*

(86) Internationale Anmeldenummer:
**PCT/EP2003/013286**

(87) Internationale Veröffentlichungsnummer:
**WO 2004/052327 (24.06.2004 Gazette 2004/26)**

(54) **NANOPARTIKUL RES REDISPERGIERBARES ZINKOXIDPULVER**

NANOPARTICULATE REDISPERSIBLE ZINC-OXIDE POWDER

POUDRE D'OXYDE DE ZINC NANOPARTICULAIRE REDISPERSIBLE

(84) Benannte Vertragsstaaten:
**AT BE BG CH CY CZ DE DK EE ES FI FR GB GR HU IE IT LI LU MC NL PT RO SE SI SK TR**

(30) Priorität: **06.12.2002 DE 10257388**

(43) Veröffentlichungstag der Anmeldung:
**31.08.2005 Patentblatt 2005/35**

(73) Patentinhaber: **Sus Tech GmbH & Co. KG**
**64287 Darmstadt (DE)**

(72) Erfinder:
- **ELSÄSSER, Ralf**
  **86153 Augsburg (DE)**
- **KLISS, Rainer**
  **64354 Reinheim (DE)**
- **HAHN, Horst**
  **64342 Seeheim-Jugenheim (DE)**
- **KROPF, Christian**
  **40724 Hilden (DE)**
- **BERBER, Mete**
  **64372 Ober-Ramstadt (DE)**
- **BULTO CARULLA, Victor**
  **08034 Barcelona (ES)**

(74) Vertreter: **Kluschanzoff, Harald**
**c/o Henkel KGaA**
**VTP Patente**
**40191 Düsseldorf (DE)**

(56) Entgegenhaltungen:

| | |
|---|---|
| **EP-A- 0 433 086** | **EP-A- 0 768 277** |
| **EP-A- 0 992 455** | **EP-A- 1 123 697** |
| **EP-A- 1 145 704** | **EP-A- 1 508 322** |
| **WO-A-02/49684** | **WO-A-03/053398** |
| **US-A- 1 997 925** | **US-A- 6 136 048** |

Bemerkungen:
Die Akte enthält technische Angaben, die nach dem Eingang der Anmeldung eingereicht wurden und die nicht in dieser Patentschrift enthalten sind.

**EP 1 567 121 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Offenbart wird ein oberflächenmodifiziertes nanoskaliges Zinkoxid, wobei die Oberflächenmodifikation eine Beschichtung mit einer organischen Säure der allgemeinen Formel $HOOC-R_1-(CH_2)_n-R_2-CH_3$, mit $R_1=CH_2-(O-CH_2-CH_2)_m$; mit m=0 bis 11, n=0 bis 30 wobei wenn m=0 ist, n größer als 11 ist und $R_2=CH_2$, $CHCH_3$, $C(CH_3)_2$, Phenylen, O, S. Dieses oberflächenmodifizierte Zinkoxid zeichnet sich dadurch aus, daß es in einem flüssigen Medium stabile Dispersionen bildet. Weiterhin betrifft die vorliegende Erfindung ein Verfahren zur Herstellung von oberflächenmodifiziertem Zinkoxid sowie ein Verfahren zur Herstellung von nanoskaligen Zinkoxiddispersionen. Derartige Zinkoxide oder Zinkoxiddispersionen können unterschiedliche technische Verwendung finden wie z.B. den Einsatz in kosmetischen Rezepturen, als UV-Schutz oder als antimikrobieller Wirkstoff.

Bekannt ist die Herstellung von Zinkoxid durch trockene und nasse Verfahren. Die klassische Methode der Verbrennung von Zink, die als trockenes Verfahren bekannt ist (z.B. Gmelin Band 32, 8. Aufl., Ergänzungsband, S. 772 ff.), erzeugt aggregierte Partikel mit einer breiten Größenverteilung. Zwar ist es grundsätzlich möglich, durch Mahlverfahren Teilchengrößen im Submikrometerbereich herzustellen, doch aufgrund der zu geringen erzielbaren Scherkräfte sind aus solchen Pulvern Dispersionen mit mittleren Teilchengrößen im unteren Nanometerbereich nicht erzielbar. Besonders feinteiliges Zinkoxid wird vor allem naßchemisch durch Fällungsprozesse hergestellt. Die Fällung in wässriger Lösung liefert in der Regel hydroxid- und/oder carbonathaltige Materialien, die thermisch zu Zinkoxid umgesetzt werden müssen. Die thermische Nachbehandlung wirkt sich dabei auf die Feinteiligkeit negativ aus, da die Partikel dabei Sinterprozessen unterworfen sind, die zur Bildung mikrometergroßer Aggregate führen, die durch Mahlung nur unvollständig auf die Primärpartikel heruntergebrochen werden können.

Nanopartikuläre Metalloxide können beispielsweise durch das Mikroemulsionsverfahren erhalten werden. Bei diesem Verfahren wird eine Lösung eines Metallalkoxids zu einer Wasser-in-Öl-Mikroemulsion getropft. In den inversen Micellen der Mikroemulsion, deren Größe im Nanometerbereich liegt, findet dann die Hydrolyse der Alkoxide zum nanopartikulären Metalloxid statt. Die Nachteile dieses Verfahrens liegen insbesondere darin, daß die Metallalkoxide teure Ausgangsstoffe darstellen, daß zusätzlich Emulgatoren verwendet werden müssen und daß die Herstellung der Emulsionen mit Tröpfchengrößen im Nanometerbereich einen aufwendigen Verfahrensschritt darstellt.

In der DE 199 07 704 wird ein nanoskaliges über eine Fällungsreaktion hergestelltes Zinkoxid beschrieben. Hierbei wird das nanoskalige Zinkoxid ausgehend von einer Zinkacetatlösung über eine alkalische Fällung hergestellt. Das abzentrifugierte Zinkoxid kann durch Zugabe von Methylenchlorid zu einem Sol redispergiert werden. Die so hergestellten Zinkoxiddispersionen haben den Nachteil, daß sie aufgrund fehlender Oberflächenmodifizierung keine gute Langzeitstabilität besitzen.

WO 02/49 684 A beschreibt die Oberflächenmodifizierung von nanoskaligem Zinkoxid mit stearinsaüre.

Der vorliegenden Erfindung lag daher die Aufgabe zugrunde, ein nanoskaliges Zinkoxid bereitzustellen, das die Herstellung stabiler nanopartikulärer Dispersionen in wenig polaren und unpolaren organischen Lösemitteln erlaubt. Um einen Einsatz in beispielsweise kosmetischen Rezepturen in technischem Maßstab realisieren zu können, ist es hierzu notwendig, von kommerziell verfügbaren, kostengünstigen Edukten für die Herstellung auszugehen, wobei das Herstellungsverfahren im weiteren eine leichte Abtrennung von Nebenprodukten ermöglichen soll. Eine irreversible Aggregation der Partikel soll nach Möglichkeit vermieden werden, damit ein aufwendiger Mahlprozeß vermieden werden kann.

Der Erfindung liegt die Erkenntnis zugrunde, daß durch eine Oberflächenmodifikation von Zinkoxid mit bestimmten langkettigen organischen Säuren eine Langzeitstabilität von Dispersionen des oberflächenmodifizierten Zinkoxids in wenig polaren und unpolaren organischen Lösemittel erreicht werden kann.

Gegenstand der vorliegenden Offenbarung ist daher ein oberflächenmodifiziertes nanopartikuläres Zinkoxid, das dadurch gekennzeichnet ist, daß die Oberflächenmodifikation eine Beschichtung mit einer organischen Säure der allgemeinen Formel $HOOC-R_1-(CH_2)_n-R_2-CH_3$ umfaßt, mit $R_1 = CH_2-(O-CH_2-CH_2)_m$; mit m = 0 bis 11, n = 0 bis 30 wobei wenn m = 0 ist n größer als 11 ist und $R_2 = CH_2$, $CHCH_3$, $C(CH_3)_2$, Phenylen, O, S.

Überraschenderweise bildet dieses oberflächenmodifizierte nanopartikuläre Zinkoxid in einem flüssigen Medium langzeitstabile Dispersionen. Für die Herstellung des Oberflächenmodifizierten Zinkoxids kann frei erhältliches Zinkoxidpulver eingesetzt werden, wobei die primäre Kristallitgröße im nanopartikulären Bereich liegen muß. Hierunter sind Teilchen zu verstehen, die einen volumengewichteten mittleren Kristallitdurchmesser von weniger als 1000 nm aufweisen, insbesondere Teilchen, die einen Durchmesser von weniger als 500 nm aufweisen. Die volumengewichtete mittlere Kristallitgröße ist mit Röntgenbeugungsverfahren, insbesondere über eine Scherrer-Analyse bestimmbar. Das Verfahren ist beispielsweise beschrieben in: C. E. Krill, R. Birringer: "Measuring average grain sizes in nanocrystalline materials", Phil. Mag. A 77, S. 621 (1998). Demnach kann die volumengewichtete mittlere Kristallitgröße D bestimmt werden durch den Zusammenhang

$$D = K\lambda/ß\cos\theta.$$

Dabei ist $\lambda$ die Wellenlänge der verwendeten Röntgenstrahlung, $\beta$ ist die volle Breite auf halber Höhe des Reflexes an der Beugungsposition $2\theta$. K ist eine Konstante der Größenordnung 1, deren genauer Wert von der Kristallform abhängt. Man kann diese Unbestimmtheit von K vermeiden, indem man die Linienverbreiterung als integrale Weite $\beta_i$ bestimmt, wobei $\beta_i$ definiert ist als die Fläche unter dem Röntgenbeugungsreflex, geteilt durch dessen maximaler Intensität $I_0$:

$$\beta_i = \frac{1}{I_0} \int_{2\theta_1}^{2\theta_2} I(2\theta) d(2\theta)$$

Dabei sind die Größen $2\theta_1$ und $2\theta_2$ die minimale und maximale Winkelposition des Bragg-Reflexes auf der $2\theta$-Achse. $I(2\theta)$ ist die gemessene Intensität des Reflexes als Funktion von $2\theta$. Unter Verwendung von diesem Zusammenhang ergibt sich als Gleichung zur Bestimmung der volumengewichteten mittleren Kristallitgröße D:

$$D = \lambda/ß_i\cos\theta$$

Dieses Zinkoxid kann direkt mit einer organischen Säure der oben angegebenen Formel oberflächenmodifiziert werden, oder zunächst einem Aktivierungsschritt unterzogen werden. Die Oberflächenaktivierung des Zinkoxids kann beispielsweise durch Versetzen mit einer stark verdünnten Säure oder Base erfolgen. Besonders gut geeignet ist die Verwendung von amorphen oder kristallinen Zinkoxiden, die über ein elektrochemisches Verfahren, das in der WO 00/14302 beschrieben ist, erhalten wird. Bei diesem Verfahren werden Metalle anodisch aufgelöst und an der Katodenseite als Metalloxide ausgefällt. Ermöglicht wird dies durch die Verwendung organischer Elektrolyte mit einem geringen Wassergehalt unter gleichzeitigem Zusatz von Leitsalzen. Beim Einsatz dieser Zinkoxide hat es sich als besonders vorteilhaft herausgestellt, wenn die Zinkoxide vor der Oberflächenmodifizierung nicht getrocknet werden, sondern als Zinkoxid-Suspension eingesetzt werden.

Ein weiterer Gegenstand der Erfindung ist ein Verfahren zur Herstellung von Zinkoxiddispersionen, das dadurch gekennzeichnet ist, daß oberflächenmodifiziertes Zinkoxid in ein organisches Lösemittel eingebracht und durch ein geeignetes Verfahren dispergiert wird.

Ein weiterer Gegenstand der vorliegenden Erfindung ist die Verwendung von oberflächenmodifiziertem Zinkoxid oder Zinkoxiddispersionen, die nach dem erfindungsgemäßen Verfahren hergestellt sind:

- zum UV-Schutz
- als antimikrobieller Wirkstoff

Ein weiterer Gegenstand der vorliegenden Erfindung ist ein Verfahren zur Oberflächenbeschichtung mit den erfindungsgemäß hergestellten Zinkoxiddispersionen. Gemäß einer bevorzugten Ausführungsform ist das oberflächenmodifizierte Zinkoxid in einem flüssigen Medium redispergierbar und bildet stabile Dispersionen. Dies ist besonders vorteilhaft, weil die aus dem erfindungsgemäß erhaltenen Zinkoxid hergestellten Dispersionen vor der Weiterverarbeitung nicht erneut dispergiert werden müssen, sondern direkt verarbeitet werden können.

Nach einer bevorzugten Ausführungsform der vorliegenden Erfindung ist das oberflächenmodifizierte Zinkoxid in unpolaren oder wenig polaren organischen Lösemitteln redispergierbar und bildet stabile Dispersionen. Dies ist besonders vorteilhaft, da hierdurch eine gleichmäßige Einarbeitung beispielsweise in Kunststoffe oder Folien möglich ist. Unter den wenig polaren und unpolaren organischen Lösemitteln sind im Sinne der vorliegenden Erfindungen die Lösemittel zu verstehen, deren Dipolmoment kleiner oder gleich 2,0 D ist.

Nach einer weiteren bevorzugten Ausführungsform des erfindungsgemäß oberflächenmodifizierten Zinkoxids ist das oberflächenmodifizierte Zinkoxid in unverzweigten, verzweigten oder cyclischen Alkanen und Alkenen, in Aromaten, in symmetrischen oder unsymmetrischen Ethern, in cyclischen Ethern, in halogenierten Kohlenwasserstoffen sowie in organischen Estern redispergierbar und bildet dort stabile Dispersionen. Dies ist besonders vorteilhaft, da sich hierdurch die Möglichkeit eröffnet, das erfindungsgemäße Material beispielsweise in kosmetischen Rezepturen, insbesondere in ölhaltigen kosmetischen Rezepturen einzusetzen.

Gemäß einer weiteren, besonders bevorzugten Ausführungsform des oberflächenmodifizierten Zinkoxids umfaßt die Oberflächenmodifizierung eine Beschichtung mit einer organischen Säure der allgemeinen Formel HOOC-$R_1$-$(CH_2)_n$-$R_2$-$CH_3$ umfaßt, mit $R_1$ = $CH_2$-$(O$-$CH_2$-$CH_2)_m$; mit m = 0 bis 11, n = 0 bis 30 wobei wenn m = 0 ist n größer als 11 ist und $R_2$ = $CH_2$, $CHCH_3$, $C(CH_3)_2$, Phenylen, O, S, wobei die Summe aus n und m größer oder gleich 5 ist. Dies ist besonders vorteilhaft, da langkettigere Moleküle bei der Oberflächenmodifizierung häufig günstig für eine bessere Redispergierbarkeit sind.

In der erfindungsgemäßen Ausfürhungs form des oberflächenmodifizierten Zinkoxids umfaßt die zur Oberflächenmodifizierung des Zinkoxids Isostearinsäure. Dies ist besonders vorteilhaft, da durch den Einsatz einer Verbindung dieses Typs die Redispergierbarkeit in unpolaren und wenig polaren organischen Lösemitteln verbessert wird.

Gemäß einer bevorzugten Ausführungsform des erfindungsgemäß oberflächenmodifizierten Zinkoxids besitzen die oberflächenmodifizierten Zinkoxidpartikel einen Durchmesser von 1 bis 200 nm. Dies ist besonders vorteilhaft, da innerhalb dieser Größenverteilung eine gute Redispergierbarkeit gewährleistet ist.

Gemäß einer besonders bevorzugten Ausführungsform des erfindungsgemäß oberflächenmodifizierten Zinkoxids weisen die Zinkoxidnanopartikel einen Durchmesser von 2 bis 50 nm, ganz besonders bevorzugt 3 bis 10 nm auf. Dieser Größenbereich ist besonders vorteilhaft, da nach Redispergierung von solchen Zinkoxidnanopartikeln die entstehenden Dispersionen transparent sind und somit beispielsweise bei Zugabe zu kosmetischen Rezepturen die Farbgebung nicht beeinflussen. Darüber hinaus ergibt sich hierdurch auch die Möglichkeit zum Einsatz in transparenten Folien. Wenn die Zinkoxiddispersionen als UV-Absorber eingesetzt werden sollen, ist es ratsam, Partikel mit einem Durchmesser von mehr als 5 nm einzusetzen, da unterhalb dieser Grenze eine Verschiebung der Absorptionskante in den kurzwelligen Bereich erfolgt (L. Brus, J. Phys., Chem. (1986), 90, 2555 - 2560).

Ein weiterer Gegenstand der vorliegenden Erfindung ist ein Verfahren zur Herstellung von oberflächenmodifiziertem Zinkoxid, das dadurch gekennzeichnet ist, daß man das unbehandelte Zinkoxid in einem unpolaren oder wenig polaren Lösemittel suspendiert, danach mit Isostearinsäure zur Oberflächenmodifizierung versetzt und erhitzt und anschließend das Lösemittel entfernt. Unter einem unpolaren oder wenig polaren Lösemittel sind im Sinne der Erfindung organische Lösemittel zu verstehen, die der Klasse der unverzweigten, verzweigten oder cyclischen Alkane und Alkene, Aromaten, symmetrischen oder unsymmetrischen Ether, cyclischen Ether, halogenierten Kohlenwasserstoffe sowie der Klasse der organischen Ester angehören, wobei auch Mischungen dieser Lösemittel im erfindungsgemäßen Verfahren eingesetzt werden können. Es ist vorteilhaft, wenn die im erfindungsgemäßen Verfahren eingesetzten Lösemittel einen Siedepunkt haben, der nicht oberhalb von 150°C liegt, um die Entfernung des Lösemittels technisch nicht zu aufwendig zu gestalten. Allerdings können auch Lösemittel eingesetzt werden, die einen höheren Siedepunkt besitzen. Besonders gut geeignete Lösemittel sind n-Pentan, n-Hexan, n-Heptan, n-Octan, Benzol, Toluol, o- m- p-Xylol, Ethylbenzol, Isopropylbenzol, ter-Butylbenzol, Methylethylether, Diethylether, Diisopropylether, Di-n-Butylether, Methyl-tert-Butylether, Cyclohexylmethylether, Diphenylether, Furan, Tetrahydrofuran, 1,4-Dioxan, Tetrachlormethan, Trichlormethan, Dichlormethan, Chlorpentafluorethan, 1,2-Dichlortetrafluorethan, Hexafluorethan, Pentachlorethan, 1,1,2,2,-Tetrachlorethan,1-Brom-2-Chlorethan, 1,2-Dichlorethan, 1,2-Dichlorpropan, Dimethylcarbonat und Diethylcarbonat oder Mischungen aus diesen. Der Einsatz von aliphatischen Alkoholen ist ebenfalls denkbar. Der Vorteil dieses erfindungsgemäßen Verfahrens ist es, daß als Produkt oberflächenmodifizierte Zinkoxide erhalten werden, die in einem flüssigen Medium sehr gut redispergierbar sind und stabile Dispersionen liefern.

Bei der Ausführungsform des erfindungsgemäßen Verfahrens zur Herstellung von oberflächenmodifiziertem Zinkoxid wird das suspendierte unbehandelte Zinkoxid mit Isostearinsäure versetzt und erhitzt (Verfahrensschritt b)). Dies ist besonders vorteilhaft, da bei einer Oberflächenmodifizierung mit Isostearinsäure oder einer Oberflächenmodifizierung, die Isostearinsäure umfaßt, die Redispergierbarkeit des nach diesem Verfahren hergestellten Zinkoxids vor allem in unpolaren Lösemitteln nahezu vollständig verläuft.

Nach einer besonders bevorzugten Ausführungsform des erfindungsgemäßen Verfahrens zur Herstellung von oberflächenmodifiziertem Zinkoxid wird das Lösemittel durch Verdampfen unter Normaldruck oder Unterdruck, durch Ausfrieren, Gefriertrocknen, Abfiltrieren und anschließendem Trocknen oder Trocknen bei erhöhter Temperatur bei Normaldruck oder bevorzugt bei vermindertem Druck entfernt. Dies ist besonders vorteilhaft, da hierdurch das Verfahren einerseits beschleunigt wird und andererseits ein schonender Umgang mit dem oberflächenmodifizierten, nanopartikulären Zinkoxid gewährleistet ist, sowie eine Möglichkeit der Lösemittelrückgewinnung gegeben wird.

Ein weiterer Gegenstand der vorliegenden Erfindung ist ein Verfahren zur Herstellung von Zinkoxiddispersionen, das dadurch gekennzeichnet ist, daß man ein erfindungsgemäß oberflächenmodifiziertes Zinkoxid in ein organisches Lösemittel oder in eine Mischung verschiedener organischer Lösemittel einbringt und durch ein geeignetes Verfahren dispergiert. Hierzu geeignete Verfahren können sein Rühren, Schütteln, Ultraschallbehandlung, Erwärmen und/ oder Einsatz kommerzieller Dispergierapparate wie Ultra-Turrax, Dissolver, Perlmühle.

Dies ist besonders vorteilhaft, da bei diesem Verfahren auch getrocknete, erfindungsgemäß oberflächenmodifiziertes Zinkoxidpulver eingesetzt werden können und in den angegebenen Lösemitteln bzw. Lösemittelgemischen nahezu vollständig wieder redispergiert werden. Hierdurch können bei Verwendung der gleichen Ausgangssubstanz durch Wahl des Dispergierungsmittels verschiedenartige Zinkoxiddispersionen hergestellt werden, die auf die unterschiedlichen Anwendungsgebiete abgestimmt sind.

Bei der Ausführung des erfindungsgemäßen Verfahrens zur Herstellung von Zinkoxiddispersionen wird ein organisches Lösemittel mit einem Dipolmoment 2,0 D eingesetzt, bevorzugt kleiner als 1,8 D. Im Rahmen der vorliegenden Erfindung kann es jedoch auch von Vorteil sein, Lösemittel mit einem Dipolmoment kleiner als 1,4 D, kleiner als 1,0 D, kleiner als 0,5 D bis zu Lösemitteln mit einem Dipolmoment von nahezu 0 einzusetzen. Welches Lösemittel mit welchem Dipolmoment sich am besten eignet richtet sich u.a. nach dem späteren Anwendungszweck der so hergestellten erfindungsgemäßen Zinkoxiddispersion. Dies ist besonders vorteilhaft, da durch die Wahl eines Löse- bzw. Dispergierungsmittels mit solchen Dipolmomenten einerseits die Langzeitstabilität der Dispersionen gewährleistet ist und andererseits hierdurch Zinkoxiddispersionen hergestellt werden, die sich beispielsweise zum Einsatz in Kunststoffen eignen.

Nach einer weiteren bevorzugten Ausführungsform des erfindungsgemäßen Verfahrens zur Herstellung Zinkoxiddispersionen ist das organische Lösemittel ausgewählt oder eine Mischung aus n-Pentan, n-Hexan, n-Heptan, n-Octan, Isododecan, Isohexadecan, Benzol, Toluol, o- m- p-Xylol, Ethylbenzol, Isopropylbenzol, ter-Butylbenzol, Methylethylether, Diethylether, Diisopropylether, Di-n-Butylether, Di-n-Pentylether, Di-n-Hexylether, Di-n-Heptylether, Di-n-Octylether, Methyl-tert-Butylether, Cyclohexylmethylether, Diphenylether, Furan, Tetrahydrofuran, 1,4-Dioxan, Tetrachlormethan, Trichlormethan, Dichlormethan, Chlorpentafluorethan, 1,2-Dichlortetrafluorethan, Hexafluorethan, Pentachlorethan, 1,1,2,2,-Tetrachlorethan,1-Brom-2-Chlorethan, 1,2-Dichlorethan, 1,2-Dichlorpropan, Dimethylcarbonat und/oder Diethylcarbonat. Dies ist besonders vorteilhaft, da durch dieses Verfahren Zinkoxiddispersionen in Flüssigkeiten unterschiedlicher physikalischer Eigenschaften hergestellt werden können, wodurch sich ein breites Anwendungsspektrum für weitere Verwendungen dieser Zinkoxiddispersionen ergibt. Als relevante physikalische Eigenschaften des Lösemittels kommen z.B. Dipolmoment, Siedetemperatur oder Schmelzpunkt in Frage. Für den späteren Einsatz der erfindungsgemäßen Zinkoxiddispersionen in Kosmetika z.B. zur Hautpflege oder in Sonneschutzprodukten ist es von Vorteil, daß mit Hilfe der erfindungsgemäßen oberflächenmodifizierten Zinkoxidpartikel Zinkoxiddispersionen in für kosmetische Anwendungen relevanten Substanzen redispergierbar sind (bspw. Di-n-Octylether) und dort vor allen Dingen auch langzeitstabile Dispersionen bilden. Dadurch ist die Gefahr einer Entmischung bei unsachgemäßer Lagerung oder Transport wesentlich geringer und auf mögliche Stabilisatorsubstanzen kann im wesentlichen Verzichtet werden.

Gemäß einer bevorzugten Ausführungsform der erfindungsgemäß erhaltenen Zinkoxiddispersionen haben die Dispersionen einen Gehalt an dispergiertem Zinkoxid von 0,1 bis 10 Gew.-%, besonders bevorzugt 1 bis 5 Gew.-%. Dispersionen mit einem solchen Gehalt an Zinkoxid haben den besonderen Vorteil, daß sie selbst bei widrigen äußeren Umständen stabil bleiben und es nicht zu einem Ausfallen des dispergierten Oxides kommt. Widrige Umstände sind in diesem Zusammenhang Umgebungstemperaturen in einem Bereich von mehr als +/- 10 °C Abweichung von der Raumtemperatur oder mechanische Belastungen wie Vibrationen oder Rühren.

Eine besonders bevorzugte Ausführungsform der erfindungsgemäß erhaltenen Zinkoxiddispersionen zeichnet sich dadurch aus, daß die Dispersionen weitestgehend transparent sind. Dies ist besonders vorteilhaft, da hierdurch die Zinkoxiddispersionen bei der Einarbeitung in andere Produkte wie z.B. Kunststoffformteile keinen Einfluß auf die Farbgebung nehmen. Insbesondere können die Zinkoxiddispersionen in Folien eingesetzt werden, da sie die Transparanz dieser Folien ebenfalls nicht beeinträchtigen.

Ein weiterer Gegenstand der vorliegenden Erfindung ist ein Verfahren zur Oberflächenbeschichtung mit dem erfindungsgemäß oberflächenmodifizierten Zinkoxid oder einer Dispersion hieraus das dadurch gekennzeichnet ist, daß man eine Dispersion der erfindungsgemäß oberflächenmodifizierten Zinkoxidpartikel auf die zu beschichtende Oberfläche aufträgt und das Dispergierungsmittel anschließend entfernt. Die Entfernung des Dispergierungsmittels kann auf verschiedene Arten vollzogen werden: Erwärmen, Abziehen des Dispergierungsmittels unter Vakuum evtl. bei gleichzeitigem Erwärmen, Gefriertrocknung, Lufttrocknung, Heißlufttrocknung, UV- und Infrarottrocknung oder Hochfrequenztrocknung. Das Beschichtungsverfahren kann auch mehrfach wiederholt werden, wenn dickere Schichten erwünscht sind. Der besondere Vorteil der dünneren Schichten ist die Transparenz für sichtbares Licht. So liefern dünne Zinkoxidbeschichtungen für optische Geräte wie Linsen, insbesondere auch Brillengläser, einen unsichtbaren UV-Breitbandfilter, der für sichtbares Licht zu nahezu 100% durchlässig ist und daher nicht zu Farbverschiebungen führt.

Nach einer bevorzugten Ausführungsform des erfindungsgemäßen Verfahrens zur Oberflächenbeschichtung wird der Zinkoxiddispersion vor dem Auftragen auf die zu beschichtende Oberfläche ein Dotierungsmittel zugesetzt. Als Dotierungsmittel für Zinkoxid eignen sich insbesondere Metallionen mit einem Elektron mehr oder einem Elektron weniger auf der äußeren Schale. Besonders geeignet sind Haupt- und Nebengruppenmetalle in der Oxidationsstufe +III. Ganz besonders bevorzugt sind Bor(III), Aluminium(III), Gallium(III) und Indium(III). Diese Metalle können in Form löslicher Salze der Dispersion zugesetzt werden, wobei die Wahl des Metallsalzes danach gerichtet ist, ob es sich im Dispergierungsmittel in gewünschter Konzentration löst. Hierbei kommen anorganische Salze oder auch Komplexe in Betracht, wie Carbonate, Halogenide, Salze mit EDTA, Nitrate, Salze mit EDTA, Acetylacetonat usw. Eine Dotierung mit Edelmetallen wie Palladium, Platin, Gold etc. ist ebenfalls möglich. Die Donatorenkonzentration kann bis zu 5% betragen. Dotierte Zinkoxidbeschichtungen eignen sich vorzugsweise zum Einsatz als transparente Elektroden für Liquid Crystal Displays, Flat Panel Displays, elektrochrome Fenster (schaltbare Lichtdurchlässigkeit), Photovoltaik-Solarzellen oder

beheizbare Spiegel.

Gemäß einer besonders bevorzugten Ausführungsform des erfindungsgemäßen Oberflächenbeschichtungsverfahrens wird die Zinkoxiddispersion über einen spin-coater auf das zu beschichtende Substrat aufgebracht. Dazu wird das zu beschichtende Werkstück in den Spin-Coater eingespannt, auf eine bestimmte Startdrehzahl gebracht und die Zinkoxiddispersion auf das Substrat gegeben. Alternativ kann die Dispersion auch unmittelbar vor oder exakt mit dem Einschalten des Spin-Coater auf das Substrat gebracht werden. Der Spin-Coater vollzieht dann im weiteren ein Drehzahlprogramm, das individuell auf das zu beschichtende Werkstück abzustimmen ist. Dies ist ein in der Technik üblicherweise notwendiger Anpassungsprozeß, der sich nach der Oberflächenbeschaffenheit des Substrats (Rauhigkeit, Benetzbarkeit, Größe, usw.) sowie nach den Eigenschaften des Beschichtungsmittels richtet (Viskosität, Benetzung, Dichte usw.). Die Umdrehungsgeschwindigkeiten bewegen sich üblicherweise im Bereich zwischen einigen hundert bis hin zu einigen tausend Umdrehungen pro Minute. Dabei kann durch eine Programmsteuerung die Drehzahl während des Aufschleuderns ("Auf-spinnes") variiert werden, wenn dies zu einer besseren Beschichtung führt. So kann für den Fall, daß die Dispersion auf das ruhende Substrat aufgebracht wird die Drehzahl nach Einschalten des Spin-Coater langsam gesteigert werden um eine gleichmäßigere Verteilung des Beschichtungsmittels zu erreichen.

Durch das Aufbringen der Schicht über einen Spin-Coater wird gleichzeitig ein Teil des Löse- bzw. Dispergierungsmittels durch Verdunstung entfernt; dies kann durch gleichzeitiges Anlegen von Vakuum während des Auf-spinnens noch verstärkt werden. Die Schichten, die mit Hilfe dieses Verfahrens in einem Aufschleudervorgang erzeugt werden weisen eine Dicke von etwa 20 bis 300 nm auf. Die Dicke kann über den Gehalt an Zinkoxid in der Dispersion und durch die Wahl des Lösemittels/ Dispergierungsmittels beeinflußt werden. Ein hoher Massenprozent-Gehalt an oberflächenmodifiziertem Zinkoxid erhöht die Schichtdicke bei einem Aufschleudervorgang; ebenfalls die Wahl eines Dispergierungsmittels mit einer höheren Viskosität. Diese Schichten zeichnen sich darüber hinaus durch eine sehr gleichmäßige Schichtdicke und geringe Rauhigkeit aus. So sind Schichten herstellbar, die eine Oberflächenrauhigkeit von weniger als 1 nm aufweisen. Durch die durch dieses Verfahren erzielbare geringe Oberflächenrauhigkeit und gleichmäßige Dicke weisen die Beschichtungen sehr homogene optische und elektrische Eigenschaften auf.

Die Herstellung dickerer Schichten in einem Aufschleudervorgang ist jedoch ebenfalls möglich, jedoch sind diese nicht mehr komplett transparent. So können Schichten bis zu 1,2 μm Dicke erzeugt werden. Alternativ zum Spin-Coating kann die Beschichtung jedoch auch durch Tauchung oder Aufsprühen einer Zinkoxiddispersion erreicht werden.

Gemäß einer weiteren besonders bevorzugten Ausführungsform des erfindungsgemäßen Beschichtungsverfahrens wird die bereits beschichtete Oberfläche anschließend auf eine Temperatur zwischen 100°C und 1000°C erwärmt. Dies wird vorzugsweise unter Sauerstoffausschluß oder unter reduzierender Atmophäre ($H_2$ oder $H_2$-haltig) durchgeführt. Die Temperdauer beträgt zwischen 10 Minuten und 6 Stunden. Die Erwärmung ist besonders vorteilhaft, weil damit einerseits Reste des Dispergierungsmittels entfernt werden und andererseits mögliche mechanische Spannungen in der Beschichtung ausheilen können. Werden dotierte Zinkoxidschichten hergestellt, so ist eine Erwärmung auf eine Temperatur von mehr als 300°C vorteilhaft, da ab etwa dieser Temperatur ein Inkorporieren des Dotierungsmittels in das Zinkoxidgitter in relativ kurzer Zeit vonstatten geht. Auch kann es von Vorteil sein, die Oberflächenbeschichtung höheren Temperaturen auszusetzen um bspw. das Dotierungsmittel bzw. das Anion des Dotierungsmittels zu entfernen (z.B. durch Oxidation). Beim Erhitzen unter reduzierender Atmosphäre können beispielsweise Edelmetallprecursor bis zum Metall reduziert werden.

Eine Erwärmung bis in die Nähe des Schmelzpunktes von Zinkoxid kann ebenfalls vorteilhaft sein, um die einzelnen Nanopartikel zu einer kontinuierlichen Schicht zusammenzusintern.

Nach einer weiteren besonders bevorzugten Ausführungsform des erfindungsgemäßen Oberflächenbeschichtungsverfahrens werden über dieses Verfahren elektrisch leitfähige Oberflächen und Schichten hergestellt. Die daraus resultierenden Vorteile wurden bereits weiter oben eingehend erläutert.

Ein weiterer Gegenstand der vorliegenden Offenbarung ist ein kosmetisches Mittel, das ein erfindungsgemäß oberflächenmodifiziertes Zinkoxid oder eine Zinkoxiddispersion enthält. Dies ist besonders vorteilhaft, da aufgrund der feinen Verteilung der Zinkoxidpartikel diese ihre hautberuhigende Wirkung effektiver entfalten können. Ein weiterer Vorteil liegt darin, daß beim Auftragen auf z.B. die Haut aufgrund der geringen Partikelgröße kein Reibeeffekt auftritt, sondern ein sanftes Auftragen möglich ist, was ein angenehmes Hautgefühl hervorruft.

Nach einer weiteren Ausführungsform des kosmetischen Mittels dient dieses der Pflege oder dem Schutz der Haut insbesondere zum Sonnenschutz bzw. zur Pflege bei Sonnenlichtexposition und liegt in Form einer Emulsion, einer Dispersion, einer Suspension, einer wässrigen Tensidzubereitung, einer Milch, einer Lotion, einer Creme, eines Balsams, einer Salbe, eines Gels, eines Granulats, eines Puders, eines Stiftpräparates, wie z.B. eines Lippenstifts, eines Schaums, eines Aerosols oder eines Sprays vor. Solche Formulierungen sind gut geeignet für topische Zubereitungen. Als Emulsionen kommen Öl-in-Wasser-Emulsionen und W/O-Emulsionen oder Mikroemulsionen in Frage. Dies ist besonders vorteilhaft weil durch den Einsatz in Sonneschutzmittel die UVabsorbierende und die hautberuhigende Wirkung des Zinkoxids gleichzeitig genutzt werden können. Darüber hinaus eignet sich das erfindungsgemäß oberflächenmodifizierte Zinkoxid sehr gut zum Einsatz in Sonnenschutzmittel, da die Partikel in einer Größe herstellbar sind, die sie für das menschliche Auge tranparent erscheinen lassen. Dadurch entsteht bei der Anwendung kein weißer

Schleier auf der Haut. Ein weiterer Vorteil ist die Tatsache, daß es sich bei Zinkoxid um einen UV-Breitbandfilter handelt, dessen UV-Absorptionsverhalten es erlaubt, ein Sonnenschutzmittel zu schaffen, daß keine weiteren chemischen UV-Filtersubstanzen mehr benötigt. Dadurch kann die Gefahr von Haut-Irritationen oder allergischen Reaktionen durch Zersetzungsprodukte chemischer Filter oder durch diese Substanzen selbst vermieden werden, was die allgemeine Verträglichkeit eines derart gestalteten Sonnenschutzmittels stark erhöht.

Im Regelfall wird das kosmetische Mittel zur topischen Applikation auf der Haut verwendet. Unter topischen Zubereitungen sind dabei solche Zubereitungen zu verstehen, die dazu geeignet sind, die Wirkstoffe in feiner Verteilung und bevorzugt in einer durch die Haut resobierbaren Form auf die Haut aufzubringen. Hierfür eignen sich z.B. wässrige und wässrig-alkoholische Lösungen, Sprays,

Schäume, Schaumaerosole, Salben, wässrige Gele, Emulsionen vom O/W- oder W/O-Typ, Mikroemulsionen oder kosmetische Stiftpräparate.

Nach einer bevorzugten Ausführungsform des kosmetischen Mittels enthält das Mittel einen Träger. Bevorzugt als Träger ist Wasser, ein Gas, eine Wasser-basierte Flüssigkeit, ein Öl, ein Gel, eine Emulsion oder Mikroemulsion, eine Dispersion oder eine Mischung davon. Die genannten Träger zeigen eine gute Hautverträglichkeit. Besonders vorteilhaft für topische Zubereitungen sind wässrige Gele, Emulsionen oder Mikroemulsionen.

Als Emulgatoren können nichtionogene Tenside, zwitterionische Tenside, ampholytische Tenside oder anionische Emulgatoren verwendet werden. Die Emulgatoren können in der erfindungsgemäßen Zusammensetzung in Mengen von 0,1 bis 10, vorzugsweise 1 bis 5, Gew.-%, bezogen auf die Zusammensetzung, enthalten sein.

Als nichtionogenes Tensid kann bspw. ein Tensid aus mindestens einer der folgenden Gruppen verwendet werden:

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- $C_{12/18}$-Fettsäuremono- und -diester von Anlagerungsprodukten von 1 bis 30 Mol Ethylenoxid an Glycerin;
- Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
- Alkylmono- und -oligoglycoside mit 8 bis 22 Kohlenstoffatomen im Alkylrest und deren ethoxylierte Analoga;
- Anlagerungsprodukte von 15 bis 60 Mol Ethylenoxid an Ricinusöl und/oder gehärtetes Ricinusöl;
- Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
- Anlagerungsprodukte von 2 bis 15 Mol Ethylenoxid an Ricinusöl und/oder gehärtetes Ricinusöl;
- Partialester auf Basis linearer, verzweigter, ungesättigter bzw. gesättigter $C_{6/22}$-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin oder Polyglycerin sowie
- Polyalkylenglycole
- Betaine

- Esterquats

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- oder eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethylammonium-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat.

Ebenfalls geeignete Emulgatoren sind ampholytische Tenside. Unter ampholytischen Tensiden werden solche oberflächenaktiven Verbindungen verstanden, die außer einer $C_{8/18}$-Alkyl- oder -Acylgruppe im Molekül mindestens eine freie Aminogruppe und mindestens eine -COOH- oder -$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkyl-gruppe. Besonders bevorzugte ampholytische Tenside sind das N-Kokosalkylaminopropionat, das Kokosacylaminoethylaminopropionat und das $C_{12/18}$-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind. Des weiteren können als anionische Emulgatoren Alkylethersulfate, Monoglyceridsulfate, Fettsäuresulfate, Sulfosuccinate und/oder Ethercarbonsäuren eingesetzt werden.

Als Ölkörper kommen Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen $C_6$-$C_{22}$-Fettalkoholen, Ester von verzweigten $C_6$-$C_{13}$-Carbonsäuren mit linearen $C_6$-$C_{22}$-Fettalkoholen, Ester von linearen $C_6$-$C_{22}$-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare $C_6$-$C_{22}$-Fettalkoholcarbonate, Guerbetcarbonate, Ester der Benzoesäure mit linearen und/oder verzweigten $C_6$-$C_{22}$-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht. Als Ölkörper können ferner auch Siliconverbindungen eingesetzt werden, beispielsweise Dimethylpolysiloxane, Methylphenylpolysiloxane, cyclische Silicone sowie amino-, fettsäure-, alkohol-, polyether-, epoxy-, fluor-, alkyl- und/oder glykosidmodifizierte Siliconverbindungen, die bei Raumtemperatur sowohl flüssig als auch harzförmig vorliegen können. Die Ölkörper können in den erfindungsgemäßen Mitteln in Mengen von 1 bis 90, vorzugsweise 5 bis 80, und insbesondere 10 bis 50, Gew.-% - bezogen auf die Zusammensetzung enthalten sein.

Nach einer besonders bevorzugten Ausführungsform enthält die Zusammensetzung weitere UV-Lichtschutzfilter in Form löslicher Verbindungen oder anderer Pigmente.

Obwohl es wie bereits weiter oben beschrieben möglich ist, mit Hilfe der erfindungsgemäß hergestellten Zinkoxidpartikel ein Sonnenschutzmittel zu schaffen, das gute UV-Absorptionseigenschaften ohne weitere UV-Filtersubstanzen erreicht, kann es im Einzelfall gewünscht sein, dem kosmetischen Mittel bzw. dem Sonnenschutzmittel weitere UV-Filtersubstanzen zuzusetzen. Dies kann z.B. dann erforderlich sein, wenn ein besonderer Schwerpunkt bei der Filterleistung gelegt werden soll. Der erfindungsgemäßen Zusammensetzung können ein oder mehrere weitere UV-Lichtschutzfilter zugesetzt werden.

Im Falle der löslichen Verbindungen sind unter UV-Lichtschutzfiltern organische Substanzen zu verstehen, die in der Lage sind, ultraviolette Strahlen zu absorbieren und die aufgenommene Energie in Form längerwelliger Strahlung, z.B. Wärme, wieder abzugeben. Die organischen Substanzen können öllöslich oder wasserlöslich sein.

Als öllösliche UV-B-Filter können z.B. folgende Substanzen verwendet werden:

- 3-Benzylidencampher und dessen Derivate, z.B. 3-(4-Methylbenzyliden)campher;

- 4-Aminobenzoesäurederivate, vorzugsweise 4-(Dimethylamino)benzoesäure-2-ethylhexylester, 4-(Dimethylamino) benzoesäure-2-octylester und 4-(Dimethylamino)benzoesäureamylester;

- Ester der Zimtsäure, vorzugsweise 4-Methoxyzimtsäure-2-ethylhexylester, 4-Methoxyzimtsäurepropylester, 4-Methoxyzimtsäureisoamylester, 4-Methoxyzimtsäureisopentylester, 2-Cyano-3-phenyl-zimtsäure-2-ethylhexylester

(Octocrylene);

- Ester der Salicylsäure, vorzugsweise Salicylsäure-2-ethylhexylester, Salicylsäure-4-isopropylbenzylester, Salicylsäurehomomenthylester;

- Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon;

- Ester der Benzalmalonsäure, vorzugsweise 4-Methoxybenzmalonsäuredi-2-ethylhexylester;

- Triazinderivate, wie z.B. 2,4,6-Trianilino-(p-carbo-2'-ethyl-1'-hexyloxy)-1 ,3,5-triazin (Octyltriazone) und Dioctyl Butamido Triazon (Uvasorb® HEB).

- Propan-1,3-dione, wie z.B. 1-(4-tert.Butylphenyl)-3-(4'methoxyphenyl)propan-1,3-dion.

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;

- Sulfonsäurederivate von Benzophenonen, vorzugsweise 2-Hydroxy-4-methoxybenzophenon-5-sulfonsäure und ihre Salze;

- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Besonders bevorzugt ist die Verwendung von Estern der Zimtsäure, vorzugsweise 4-Methoxyzimtsäure-2-ethylhexylester, 4-Methoxyzimtsäureiso-pentylester, 2-Cyano-3-phenyl-zimtsäure-2-ethylhexylester (Octocrylene). Des weiteren ist die Verwendung von Derivaten des Benzophenons, insbesondere 2-Hydroxy-4-methoxybenzophenon, 2-Hydroxy-4-methoxy-4'-methylbenzophenon, 2,2'-Dihydroxy-4-methoxybenzophenon sowie der Einsatz von Propan-1,3-dionen, wie z.B. 1-(4-tert.Butylphenyl)-3-(4'methoxyphenyl)propan-1,3-dion bevorzugt.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan oder 1-Phenyl-3-(4'-isopropylphenyl)-propan-1,3-dion. Die UV-A und UV-B-Filter können selbstverständlich auch in Mischungen eingesetzt werden.

Als weitere Lichtschutzfilter können aber auch andere unlösliche Pigmente, z.B. feindisperse Metalloxide bzw. Salze wie beispielsweise Titandioxid, Eisenoxid, Aluminiumoxid, Ceroxid, Zirkoniumoxid, Silicate (Talk), Bariumsulfat und Zinkstearat verwendet werden. Die Partikel sollten dabei einen mittleren Durchmesser von weniger als 100 nm, vorzugsweise zwischen 5 und 50 nm und insbesondere zwischen 15 und 30 nm aufweisen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Der Gesamtanteil der Lichtschutzmittel im Sonnenschutzmittel liegt üblicherweise bei 1 bis 20, vorzugsweise 5 bis 15 Gew.-%. Die Zusammensetzung als solche kann 1 bis 95, vorzugsweise 5 bis 80, und insbesondere 10 bis 60 Gew.-% Wasser enthalten.

Nach einer besonders bevorzugten Ausführungsform enthält die kosmetische Zusammensetzung ferner pflegende Substanzen, weitere kosmetische Wirkstoffe und/oder Hilfs- und Zusatzstoffe. Als weitere kosmetische Wirkstoffe werden insbesondere Hautfeuchthaltemittel, antimikrobielle Stoffe und/oder deodorierende oder schweißhemmende Stoffe eingesetzt. Dies hat den Vorteil, dass sich weitere gewünschte Effekte erzielen lassen, die zur Pflege oder Behandlung der Haut beitragen oder beispielsweise das Wohlempfinden des Anwenders der kosmetischen Zusammensetzung bei der Verwendung dieser Zusammensetzung steigern.

So können in der kosmetischen Zusammensetzung neben dem Träger, dem oberflächenmodifizierten Zinkoxid, Wasser und physiologisch geeigneten Lösemitteln unter anderem auch pflegende Bestandteile, wie z.B. Öle, Wachse, Fette, rückfettende Substanzen, Verdickungsmittel, Emulgatoren und Duftstoffe enthalten sein. Ein hoher Anteil an pflegenden Substanzen ist insbesondere zur topischen prophylaktischen oder kosmetischen Behandlung der Haut vorteilhaft. Besonders vorteilhaft ist es, wenn die Zusammensetzung neben den in vielen Fällen ebenfalls Pflegewirkung aufweisenden tierischen und pflanzlichen Fetten und Ölen noch weitere Pflegekomponenten enthält. Die Gruppe der

pflegenden Wirkstoffe, die zum Einsatz kommen können, umfasst z.B.: Fettalkohole mit 8-22 C-Atomen, insbesondere Fettalkohole von natürlichen Fettsäuren; tierische und pflanzliche Proteinhydrolysate, insbesondere Elastin-, Kollagen-, Keratin-, Milcheiweiß-, Sojaprotein-, Seidenprotein-, Haferprotein-, Erbsenprotein-, Mandelprotein- und Weizenproteinhydrolysate; Vitamine und Vitaminvorstufen, insbesondere die der Vitamin-Gruppen A und B; Mono-, Di- und Oligosaccharide; Pflanzenextrakte; Honigextrakte; Ceramide; Phospholipide; Vaseline, Paraffin- und Silikonöle; Fettsäure- und Fettalkoholester, insbesondere die Monoester der Fettsäuren mit Alkoholen mit 3-24 C-Atomen.

Zu den in der Zusammensetzung bevorzugt einzusetzenden Vitaminen, Provitaminen oder Vitaminvorstufen gehören unter anderem:

- Vitamine, Provitamine und Vitaminvorstufen aus den Gruppen A, C, E und F, insbesondere 3,4-Didehydroretinol (Vitamin $A_2$), β-Carotin (Provitamin des Vitamin $A_1$), Ascorbinsäure (Vitamin C), sowie die Palmitinsäureester, Glucoside oder Phosphate der Ascorbinsäure, Tocopherole, insbesondere α-Tocopherol sowie seine Ester, z. B. das Acetat, das Nicotinat, das Phosphat und das Succinat; weiterhin Vitamin F, worunter essentielle Fettsäuren, besonders Linolsäure, Linolensäure und Arachidonsäure, verstanden werden; Vitamin A und seine Derivate und Provitamine zeigen vorteilhafterweise einen besonderen hautglättenden Effekt.

Zu den in der Zusammensetzung bevorzugt einzusetzenden Vitaminen, Provitaminen oder Vitaminvorstufen der Vitamin B-Gruppe oder deren Derivaten sowie den Derivaten von 2-Furanon gehören unter anderem:

- Vitamin $B_1$, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf das gesamte Mittel, eingesetzt.
- Vitamin $B_2$, Trivialname Riboflavin, chemische Bezeichung 7,8-Dimethyl-10-(1-D-ribityl)-benzo[g]pteridin-2,4(3*H*, 10*H*)-dion. In freier Form kommt Riboflavin z. B. in Molke vor, andere Riboflavin-Derivate lassen sich aus Bakterien und Hefen isolieren. Ein erfindungsgemäß ebenfalls geeignetes Stereoisomeres des Riboflavin ist das aus Fischmehl oder Leber isolierbare Lyxoflavin, das statt des D-Ribityl einen D-Arabityl-Rest trägt. Bevorzugt werden Riboflavin oder seine Derivate in Mengen von 0,05 bis 1 Gew.-%, bezogen auf das gesamte Mittel, eingesetzt.
- Vitamin $B_3$. Unter dieser Bezeichnung werden häufig die Verbindungen Nicotinsäure und Nicotinsäureamid (Niacinamid) geführt. Bevorzugt ist das Nicotinsäureamid, das in den Mitteln bevorzugt in Mengen von 0,05 bis 1 Gew.-%, bezogen auf das gesamte Mittel, enthalten ist.
- Vitamin $B_5$ (Pantothensäure und Panthenol). Bevorzugt wird Panthenol eingesetzt. Einsetzbare Derivate des Panthenols sind insbesondere die Ester und Ether des Panthenols sowie kationisch derivatisierte Panthenole. Es können an Stelle von sowie zusätzlich zu Pantothensäure oder Panthenol auch Derivate des 2-Furanon mit der allgemeinen Strukturformel (I) eingesetzt werden.

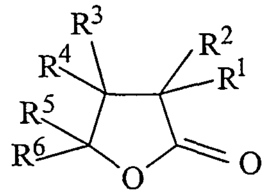

(I)

Bevorzugt sind die 2-Furanon-Derivate, in denen die Substituenten $R^1$ bis $R^6$ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten $C_2$-$C_4$ - Kohlenwasserstoffrest, einen gesättigten oder ein- oder zweifach ungesättigten, verzweigten oder linearen Mono-, Di- oder Trihydroxy-$C_2$-$C_4$ - Kohlenwasserstoffrest oder einen gesättigten oder ein- oder zweifach ungesättigten, verzweigten oder linearen Mono-, Di- oder Triamino-$C_2$-$C_4$ - Kohlenwasserstoffrest darstellen. Besonders bevorzugte Derivate sind die auch im Handel erhältlichen Substanzen

Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) $R^1$ für eine Hydroxylgruppe, $R^2$ für ein Wasserstoffatom, $R^3$ und $R^4$ für eine Methylgruppe und $R^5$ und $R^6$ für ein Wasserstoffatom stehen. Das Stereoisomer (R)-Pantolacton entsteht beim Abbau von Pantothensäure.

Vorteilhafterweise verleihen diese Verbindungen der kosmetischen Zusammensetzung feuchtigkeitsspendende sowie hautberuhigende Eigenschaften.

Die genannten Verbindungen des Vitamin $B_5$-Typs sowie die 2-Furanonderivate sind in den erfindungsgemäßen Mitteln bevorzugt in einer Gesamtmenge von 0,05 bis 10 Gew.-%, bezogen auf das gesamte Mittel, enthalten. Gesamtmengen von 0,1 bis 5 Gew.-% sind besonders bevorzugt.

- Vitamin $B_6$, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin $B_6$ ist in den erfindungsgemäßen Mitteln bevorzugt in Mengen von 0,0001 bis 1,0 Gew.-%, insbesondere in Mengen von 0,001 bis 0,01 Gew.-%, enthalten.

- Vitamin $B_7$ (Biotin), auch als Vitamin H oder "Hautvitamin" bezeichnet. Bei Biotin handelt es sich um (3aS,4S, 6a*R*)-2-Oxohexahydrothienol[3,4-*d*]-imidazol-4-valeriansäure. Biotin ist in den Mitteln bevorzugt in Mengen von 0,0001 bis 1,0 Gew.-%, insbesondere in Mengen von 0,001 bis 0,01 Gew.-% enthalten.

Panthenol, Pantolacton, Nicotinsäureamid sowie Biotin sind ganz besonders bevorzugt.

Unter Hilfs- und Zusatzstoffen sind Stoffe zu verstehen, die zur Verbesserung der ästhetischen, anwendungstechnischen und/oder kosmetischen Eigenschaften geeignet sind, wie z.B. Co-Emulgatoren, organische Lösemittel, Überfettungsmittel, Stabilisatoren, Antioxidationsmittel, Wachse oder Fette, Konsistenzgeber, Verdickungsmittel, Bräunungsmittel, Vitamine, kationische Polymere, biogene Wirkstoffe, Konservierungsmittel, Hydrotope, Solubilisatoren, Farb- und Duftstoffe.

Beispielsweise können folgende Hilfs- und Zusatzstoffe verwendet werden:

- Allantoin,

- Aloe Vera,

- Bisabolol,

- Ceramide und Pseudoceramide,

- Antioxidationsmittel verbessern vorteilhafterweise die Stabilität der erfindungsgemäßen Zusammensetzungen. Antioxidantien sind beispielsweise Aminosäuren (z. B. Glycin, Histidin, Tyrosin, Tryptophan) und deren Derivate, Imidazol und Imidazolderivate (z. B. Urocaninsäure), Peptide wie z. B. D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis μmol/kg), ferner Metallchelatoren (z. B. α-Hydroxyfettsäuren, EDTA, EGTA, Phytinsäure, Lactoferrin), α-Hydroxysäuren (z. B. Zitronensäure, Milchsäure, Äpfelsäure), Huminsäuren, Gallensäure, Gallenextrakte, Gallussäureester (z. B. Propyl-, Octyl- und Dodecylgallat), Flavonoide, Catechine, Bilirubin, Biliverdin und deren Derivate, ungesättigte Fettsäuren und deren Derivate (z. B. γ-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat, -stearat, -dipalmitat, -acetat, Mg-Ascorbylphosphate, Natrium- und Magnesiumascorbat, Dinatriumascorbylphosphat und -sulfat, Kaliumascorbyltocopherylphosphat, Chitosanascorbat), Isoascorbinsäure und deren Derivate, Tocopherole und deren Derivate (z. B. Tocopherylacetat, -linoleat, -oleat und - succinat, Tocophereth-5, Tocophereth-10, Tocophereth-12, Tocophereth-18, Tocophereth-50, Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat,

Zimtsäure und deren Derivate (z. B. Ferulasäure, Ethylferulat, Kaffeesäure), Kojisäure, Chitosanglycolat und - salicylat, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und Zink-Derivate (z. B. ZnO, $ZnSO_4$), Selen und Selen-Derivate (z. B. Selenmethionin), Stilbene und Stilben-Derivate (z. B. Stilbenoxid, trans-Stilbenoxid). Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte (z. B. Teebaumöl, Rosmarinextrakt und Rosmarinsäure), die diese Antioxidantien enthalten, eingesetzt werden. Als lipophile, öllösliche Antioxidantien aus dieser Gruppe sind Tocopherol und dessen Derivate, Gallussäureester, Flavonoide und Carotinoide sowie Butylhydroxytoluol/anisol bevorzugt. Als wasserlösliche Antioxidantien sind Aminosäuren, z. B. Tyrosin und Cystein und deren Derivate sowie Gerbstoffe, insbesondere solche pflanzlichen Ursprungs bevorzugt. Die Gesamtmenge der Antioxidantien in den kosmetischen Zusammensetzungen beträgt 0,001 - 20 Gew.-%, vorzugsweise 0,05 - 10 Gew.-%, insbesondere 0,1 - 5 Gew.-% und ganz besonders bevorzugt 0,1 bis 2 Gew.-%.

- Triterpene, insbesondere Triterpensäuren wie Ursolsäure, Rosmarinsäure, Betulinsäure, Boswelliasäure und Bryonolsäure,

- Monomere Catechine, besonders Catechin und Epicatechin, Leukoanthocyanidine, Catechinpolymere (Catechin-Gerbstoffe) sowie Gallotannine,

- Verdickungsmittel, z. B. Gelatine, Pflanzengumme wie Agar-Agar, Guar-Gum, Alginate, Xanthan-Gum, Gummi arabicum, Karaya-Gummi oder Johannisbrotkernmehl, natürliche und synthetische Tone und Schichtsilikate, z. B. Bentonit, Hectorit, Montmorillonit oder Laponite®, vollsynthetische Hydrokolloide wie z. B. Polyvinylalkohol, und außerdem Ca-, Mg- oder Zn-Seifen von Fettsäuren,

- Pflanzenglycoside,

- Strukturanten wie Maleinsäure und Milchsäure,

- Dimethylisosorbid,

- Alpha-, beta- sowie gamma-Cyclodextrine, insbesondere zur Stabilisierung von Retinol,

- Lösemittel, Quell- und Penetrationsstoffe wie Ethanol, Isopropanol, Ethylenglykol, Propylenglykol, Propylenglykolmonoethylether, Glycerin und Diethylenglykol, Carbonate, Hydrogencarbonate, Guanidine, Harnstoffe sowie primäre, sekundäre und tertiäre Phosphate

- Parfümöle, Pigmente sowie Farbstoffe zum Anfärben des Mittels,

- Substanzen zur Einstellung des pH-Wertes, z. B. $\alpha$- und $\beta$-Hydroxycarbonsäuren,

- Komplexbildner wie EDTA, NTA, $\beta$-Alanindiessigsäure und Phosphonsäuren,

- Trübungsmittel wie Latex, Styrol/PVP- und Styrol/Acrylamid-Copolymere,

- Perlglanzmittel wie Ethylenglykolmono- und -distearat sowie PEG-3-distearat,

- Treibmittel wie Propan-Butan-Gemische, $N_2O$, Dimethylether, $CO_2$ und Luft.

Die Zugabe von Allantoin, Bisabolol und/oder Aloe Vera auch in Form von Extrakten zu den erfindungsgemäßen kosmetischen Zusammensetzungen verbessert die weiterhin die hautberuhigenden, feuchtigkeitsspendenden und hautpflegende Eigenschaften der Formulierungen und ist daher besonders bevorzugt.

Als weitere Inhaltsstoffe kann die kosmetische Zusammensetzung in untergeordneten Mengen weitere, mit den anderen Inhaltsstoffen kompatible Tenside enthalten. Typische Beispiele für anionische Tenside sind Seifen, Alkylbenzolsulfonate, Alkansulfonate, Olefinsulfonate, Alkylethersulfonate, Glycerinethersulfonate, $\alpha$-Methylestersulfonate, Sulfofettsäuren, Alkylsulfate, Fettalkoholethersulfate, Glycerinethersulfate, Hydroxymischethersulfate, Monoglycerid (ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether) phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk (en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine.

Nach einer weiteren besonders bevorzugten Ausführungsform wird das kosmetische Mittel als Sonnenschutzmittel verwendet. Die daraus resultierenden Vorteile wurden bereits eingehend erläutert. In Beispiel 4 ist eine Basisrezeptur mit Einsatz des erfindungsgemäß beschichteten Zinkoxids (mit Isostearinsäure) beschrieben.

Der Einsatz der Zinkoxiddispersionen ist insbesondere ebenfalls möglich in Haarkosmetika wie Shampoos, Conditioner, Spülungen, Haarwässer, Haargel, Haarspray etc.. Insbesondere leave-on Produkte, die nach erfolgter Applikation auf dem Haar bzw. der Kopfhaut verbleiben sind besonders gut geeignet. Das so auf die Kopfhaut und das Haar aufgetragene Zinkoxid kann somit auch dort als UV-Schutzmittel wirken bzw. auf der Kopfhaut seine hautberuhigende Wirkung entfalten.

Nach einer bevorzugten Ausführungsform des kosmetischen Mittels wird das kosmetische Mittel auf die Oberfläche des zu behandelnden bzw. zu schützenden Körpers also topisch aufgetragen. Diese Applikationsform ist besonders vorteilhaft, da sie einfach zu handhaben ist, so dass Fehldosierungen weitestgehend ausgeschlossen sind. Ferner lässt sich ein zusätzlicher pflegender Effekt für die Haut erreichen. Falls nur einzelne Körperteile der Sonnenstrahlung ausgesetzt werden, kann das Sonnenschutzmittel außerdem nur gezielt auf diese Körperteile aufgetragen werden.

Ein weiterer Gegenstand der vorliegenden Erfindung ist die Verwendung von erfindungsgemäß oberflächenmodiziertem Zinkoxid oder Zinkoxiddispersionen zum UV-Schutz. Dies ist besonders vorteilhaft, da aufgrund der Feinteiligkeit des oberflächenmodifizierten Zinkoxids und der guten Verteilung eine besonders hohe UV-Absorption erreicht wird.

Ein weiterer Gegenstand der vorliegenden Erfindung ist die Verwendung von erfindungsgemäß oberflächenmodiziertem Zinkoxid oder Zinkoxiddispersionen als antimikrobieller Wirkstoff. Die Verwendung dieser Partikel ist für diesen Einsatzzweck besonders vorteilhaft, da aufgrund der Feinteiligkeit der Partikel und der daraus resultierenden großen Oberfläche, die antimikrobielle Wirkung stark verbessert ist und andererseits aufgrund der guten Dispergiereigenschaften des Materials das Zinkoxid in fein verteilter Form vorliegt. Somit kann das Zinkoxid problemlos in verschiedenen Darreichungsformen eingesetzt werden wie beispielsweise Cremes, Hautmilch, Lotionen oder Tonics.

Ein weiterer Gegenstand der vorliegenden Offenbarung ist ein pharmazeutisches Mittel, das ein erfindungsgemäß oberflächenmodifiziertes Zinkoxid oder eine Zinkoxiddispersion enthält. Dieses pharmazeutische Mittel weist sich dadurch aus, daß aufgrund der Feinteiligkeit der Partikel die pharmazeutische Wirksamkeit stark erhöht ist. Darüber hinaus besitzt das pharmazeutische Mittel den Vorteil, daß aufgrund der bereits oben beschriebenen guten Langzeitstabilität der Zinkoxiddispersionen auf den Zusatz von Stabilisatoren verzichtet werden kann, die eine Entmischung verhindern. Somit wird zusätzlich die Verträglichkeit des pharmazeutischen Mittels erhöht.

**Beispiel 1:**

Fällung:

In einem heiz- und kühlbaren 20l-Reaktor mit Innenthermometer und mechanischem Rührer werden 1110g $ZnCl_2$ (8,15 mol) in 11,1 l Methanol aufgelöst. Zu dieser Lösung werden 651 g NaOH-Plätzchen (16,3 mol) in 2 Portionen bei Raumtemperatur gegeben. Durch die NaOH-Zugabe steigt die Temperatur der Lösung an und ein weißer Niederschlag (NaCl / ZnO) fällt aus. Danach wird noch ca. 1 Stunde gerührt, wobei die Temperatur wieder auf Raumtemperatur absinkt. Anschließend wird die feste Phase abfiltriert und danach zentrifugiert.

Modifizierung:

Der weiße Rückstand wird 2 Mal mit THF gewaschen und danach erneut in den Reaktor überführt und in 11 l THF suspendiert. Zu dieser Suspension werden 385 g Isostearinsäure gegeben. Anschließend wird die Suspension 3 Stunden unter Rückfluß gekocht. Danach läßt man die Lösung ruhen, wobei sich das NaCl absetzt. Die das oberflächenmodifizierte Zinkoxid enthaltene überstehende nahezu klare Phase wird abgesaugt, der feste Rückstand (NaCl)

wird verworfen. Danach wird das Lösemittel abdestilliert und das Rohprodukt in 3 l Toluol aufgenommen. Zur Entfernung der NaCl-Reste wird die Lösung zentrifugiert. Die abzentrifugierte Lösung wird mit 4 l Ethanol (96%) versetzt, das ausfallende weiße oberflächenmodifizierte Zinkoxid abzentrifugiert, im Vakuum getrocknet und anschließend in einer Mörsermühle fein zerkleinert. Man erhält 784 g isostearinsäurebeschichtetes Zinkoxid.

Redispergierung:

Das Produkt wird in 3 gleich große Portionen aufgeteilt und durch einfaches Aufrühren in jeweils 1,5 l Cyclohexan, Heptan und Toluol ohne Einsatz von mechanischen Dispergierhilfen (Dissolver, Ultra-Turrax) oder Behandlung mit Ultraschall auf Primärpartikelgröße redispergiert. Die entstehenden Dispersionen sind nahezu transparent.

**Beispiel 2:**

Herstellung einer Zinkoxid-Schicht auf einem Glassubstrat.

Auf einen runden Glaswafer (Corning 1737 F) werden 500 μl einer 20% igen Dispersion von mit Isostearinsäure oberflächenmodifiziertem Zinkoxid in Cyclohexan mit dem Einschalten des Spin-Coaters gleichmäßig aufgebracht. Das Drehzahlprogramm ist derart eingestellt, daß die Drehzahl 5 s bei 500 $min^{-1}$ verweilt, während die Dispersion auf das Substrat gespritzt wird. Danach wird die Drehzahl über ein Intervall von 10 s auf 4000 $min^{-1}$ erhöht und diese Drehzahl für 20 s beibehalten. Anschließend wird wieder auf 0 $min^{-1}$ abgebremst. Die in Fig. 1 gezeigte REM Aufnahme zeigt einen mittleren Teilchendurchmesser von etwa 8 nm.

Danach wird das beschichtete Substrat im Muffelofen bei 450°C für eine Stunde in Luft-Atmosphäre getempert (dT/dt=1 °C/min). Die in Fig. 2 gezeigte REM-Aufnahme zeigt einen mittleren Teilchendurchmesser nach dem Tempern von etwa 40 nm.

Die Schichtdicke wird mit Hilfe eines Profilometers zu 200 nm bestimmt. Die Auswertung der mittleren Teilchengröße über die statistische Auswertung einer REM-Aufnahme liefert einen Wert 30 nm. Die Transmission liegt im visuellen Bereich (400-800 nm) bei 98,9%. Der spezifische elektrische Widerstand wurde über eine 4-Punkt Messung zu $1{,}1 \cdot 10^4$ Ωcm bestimmt.

**Beispiel 3:**

0,5 g mit Isostearinsäure oberflächenmodifiziertes Zinkoxid (mittlere Kristallitgröße 9 nm) werden in 5 ml Toluol unter Rühren dispergiert und 0,028 g Aluminiumacetylacetonat hinzugegeben. Die transparente Dispersion wird durch einen 0,2 μm Filter filtriert. 700 μl dieser Dispersion werden analog Beispiel 2 an einem Spin-Coater auf das Glassubstrat (Corning 1737 F) aufgebracht und anschließend in einem Rohrofen in einer Atmosphäre von 5% $H_2$ 95% $N_2$ bei 450 °C für eine Stunde getempert.

Der spezifische elektrische Widerstand wurde über eine 4-Punkt Messung zu $1{,}2 \cdot 10^{-1}$ Ωcm bestimmt.

**Beispiel 4:**

Die folgenden Komponenten wurden miteinander zu einem Sonnenschutzmittel verarbeitet:

| Bestandteil | Gew.% |
|---|---|
| Cetiol OE (Di-n-octylether) | 10 |
| Cetiol S (Diisooctylcyclohexan) | 10 |
| Lanette O (Cetylstearylalkohol) | 4,5 |
| Eumulgin B2 (Polyoxyethylen-20-Cetylstearylalkohol | 2 |
| Monomuls 60-35 C (Monoglycerid der Palmfettsäure, gehärtet) | 2 |
| Baysilon M 350 (Silikonöl) | 0,5 |
| Phenonip (Konservierungsmittel) | 1 |
| Zinkoxid (mit Isostearinsäure oberflächenmodifiziert) | 8 |
| Zinkoxid (mit Polyglykoldisäure oberflächenmodifiziert) | 8 |
| Destilliertes Wasser | Ad 100 |

Herstellung Komponente A:

Die zur Herstellung eingesetzte Apparatur besteht aus einem Becherglas, Propellerrührer und einem Silikonbad mit Heizplatte. Die Emulgatoren und das Baysilon-Öl werden im Becherglas vorgelegt, auf 85-90°C aufgeheizt. Das hydrophobe oberflächenmodifizierte Zinkoxid (mit Isostearinsäure oberflächenmodifiziert) wird mittels Magnetrührer in einem der kosmetischen Öle oder in einem Gemisch der kosmetischen Öle (Cetiol) dispergiert. Anschließend wird die Zinkoxid-Dispersion zu der Emulgatorschmelze gegeben. Danach wird das auf 85-100°C vorgeheizte Wasser hinzugegeben. Die Mischung wird ca. 10 Minuten bei 85-90°C nachgerührt und anschließend abgekühlt. Während des Abkühlens wurde bei ca. 40°C das Konservierungsmittel hinzugegeben und kräftig untergerührt.

Herstellung einer Sonnencreme

Zur Herstellung der Sonnencreme wird zunächst die oben angegebene Menge an hydrophil oberflächenbeschichtetem Zinkoxid (mit Polyglykoldisäure oberflächenmodifiziert) in der angegebenen Menge Wasser unter Rühren dispergiert und auf 40°C erwärmt. Zu dieser Dispersion wird nun die Komponente A unter kräftigem Rühren hinzugegeben. Anschließend wurde die Creme unter Rühren abgekühlt.

## Patentansprüche

1. Verfahren zur Herstellung von oberflächenmodifiziertem nanopartikulärem Zinkoxid, **dadurch gekennzeichnet, dass**

    a) unbehandeltes Zinkoxid in einem unpolaren oder wenig polaren Lösemittel mit einem Dipolmoment kleiner oder gleich 2,0 D suspendiert wird,
    b) danach mit Isostearinsäure versetzt und erhitzt wird und
    c) das Lösemittel entfernt wird.

2. Verfahren nach Anspruch 1, **dadurch gekennzeichnet, dass** die Zinkoxid-Primärteilchen einen Durchmesser von 1 bis 200 nm besitzen.

3. Verfahren nach Anspruch 2, **dadurch gekennzeichnet, dass** die Zinkoxid-Primärteilchen einen Durchmesser von 2-50 nm, insbesondere 3-10 nm, aufweisen.

4. Verfahren nach Anspruch 1 bis 3, **dadurch gekennzeichnet, dass** das Lösemittel ein Dipolmoment kleiner oder gleich 1,8 D aufweist.

5. Verfahren nach einem der Ansprüche 1 bis 4, **dadurch gekennzeichnet, dass** das Lösemittel ausgewählt ist aus unverzweigten, verzweigten oder cyclischen Alkanen und Alkenen, Aromaten, symmetrischen oder unsymmetrischen Ethern, cyclischen Ethern, halogenierten Kohlenwasserstoffen sowie organischen Estern oder Mischungen dieser Lösemittel.

6. Verfahren nach Anspruch 5, **dadurch gekennzeichnet, dass** das Lösemittel ausgewählt ist aus n-Pentan, n-Hexan, n-Heptan, n-Octan, Benzol, Toluol, o- m- p-Xylol, Ethylbenzol, Isopropylbenzol, ter-Butylbenzol, Methylethylether, Diethylether, Diisopropylether, Di-n-Butylether, Methyl-tert-Butylether, Cyclohexylmethylether, Diphenylether, Furan, Tetrahydrofuran, 1,4-Dioxan, Tetrachlormethan, Trichlormethan, Dichlormethan, Chlorpentafluorethan, 1,2-Dichlortetrafluorethan, Hexafluorethan, Pentachlorethan, 1,1,2,2,-Tetrachlorethan,1-Brom-2-Chlorethan, 1,2-Dichlorethan, 1,2-Dichlorpropan, Dimethylcarbonat und Diethylcarbonat oder Mischungen dieser Lösemittel.

7. Verfahren nach einem der Ansprüche 1 bis 6, **dadurch gekennzeichnet, dass** das Lösemittel durch Verdampfen unter Normaldruck oder Unterdruck, durch Ausfrieren, Gefriertrocknen, Abfiltrieren und anschließendem Trocknen oder Trocknen bei erhöhter Temperatur bei Normaldruck oder bevorzugt bei vermindertem Druck entfernt wird.

8. Verwendung des nach den Ansprüchen 1 bis 7 hergestellten oberflächenmodifizierten Zinkoxids zur Herstellung stabiler Zinkoxiddispersionen in organischen Lösemitteln, die ein Dipolmoment kleiner oder gleich 2,0 D aufweisen.

9. Verwendung von Zinkoxiddispersionen nach Anspruch 8 zur Beschichtung von Oberflächen.

**10.** Verwendung des nach den Ansprüchen 1 bis 7 hergestellten oberflächenmodifizierten Zinkoxids in kosmetischen Mitteln zur Pflege oder zum Schutz der Haut, insbesondere zum Sonnenschutz.

**11.** Verwendung des nach den Ansprüchen 1 bis 7 hergestellten oberflächenmodifizierten Zinkoxids als antimikrobieller Wirkstoff.

**12.** Verwendung des nach den Ansprüchen 1 bis 7 hergestellten oberflächenmodifizierten Zinkoxids in pharmazeutischen Mitteln.

**Claims**

**1.** Process for the preparation of surface-modified nanoparticulate zinc oxide, **characterized in that**

a) untreated zinc oxide is suspended in a nonpolar or low-polarity solvent with a dipole moment of less than or equal to 2.0 D,
b) then admixed with isostearic acid and heated and
c) the solvent is removed.

**2.** Process according to Claim 1, **characterized in that** the zinc oxide primary particles have a diameter of from 1 to 200 nm.

**3.** Process according to Claim 2, **characterized in that** the zinc oxide primary particles have a diameter of 2-50 nm, in particular 3-10 nm.

**4.** Process according to Claim 1 to 3, **characterized in that** the solvent has a dipole moment of less than or equal to 1.8 D.

**5.** Process according to one of Claims 1 to 4, **characterized in that** the solvent is selected from unbranched, branched or cyclic alkanes and alkenes, aromatics, symmetrical or asymmetrical ethers, cyclic ethers, halogenated hydrocarbons, and organic esters or mixtures of these solvents.

**6.** Process according to Claim 5, **characterized in that** the solvent is selected from n-pentane, n-hexane, n-heptane, n-octane, benzene, toluene, o-, m-, p-xylene, ethylbenzene, isopropylbenzene, tert-butylbenzene, methyl ethyl ether, diethyl ether, diisopropyl ether, di-n-butyl ether, methyl tert-butyl ether, cyclohexyl methyl ether, diphenyl ether, furan, tetrahydrofuran, 1,4-dioxane, tetrachloromethane, trichloromethane, dichloromethane, chloropentafluoroethane, 1,2-dichlorotetrafluoroethane, hexafluoroethane, pentachloroethane, 1,1,2,2-tetrachloroethane, 1-bromo-2-chloroethane, 1,2-dichloroethane, 1,2-dichloropropane, dimethyl carbonate and diethyl carbonate or mixtures of these solvents.

**7.** Process according to one of Claims 1 to 6, **characterized in that** the solvent is removed by evaporation under atmospheric pressure or subatmospheric pressure, by freezing, freeze-drying, filtering and subsequent drying or drying at elevated temperature at atmospheric pressure or preferably at reduced pressure.

**8.** Use of the surface-modified zinc oxide prepared according to Claims 1 to 7 for preparing stable zinc oxide dispersions in organic solvents which have a dipole moment of less than or equal to 2.0 D.

**9.** Use of zinc oxide dispersions according to Claim 8 for the coating of surfaces.

**10.** Use of the surface-modified zinc oxide prepared according to Claims 1 to 7 in cosmetic compositions for the care or protection of the skin, in particular for sun protection.

**11.** Use of the surface-modified zinc oxide prepared according to Claims 1 to 7 as antimicrobial active ingredient.

**12.** Use of the surface-modified zinc oxide prepared according to Claims 1 to 7 in pharmaceutical compositions.

## Revendications

**1.** Procédé pour la préparation d'oxyde de zinc nanoparticulaire dont la surface a été modifiée, **caractérisé en ce que**

a) on met en suspension de l'oxyde de zinc non traité dans un solvant apolaire ou peu polaire, possédant un moment dipolaire inférieur ou égal à 2,0 D ;
b) on ajoute ensuite de l'acide isostéarique et on chauffe ; et
c) on élimine le solvant.

**2.** Procédé selon la revendication 1, **caractérisé en ce que** les particules primaires d'oxyde de zinc possèdent un diamètre de 1 à 200 nm.

**3.** Procédé selon la revendication 2, **caractérisé en ce que** les particules primaires d'oxyde de zinc possèdent un diamètre de 2 à 50 nm, en particulier de 3 à 10 nm.

**4.** Procédé selon les revendications 1 à 3, **caractérisé en ce que** le solvant présente un moment dipolaire inférieur ou égal à 1,8 D.

**5.** Procédé selon l'une quelconque des revendications 1 à 4, **caractérisé en ce que** le solvant est choisi parmi des alcanes et des alcènes non ramifiés, ramifiés ou cycliques, des composés aromatiques, des éthers symétriques ou asymétriques, des éthers cycliques, des hydrocarbures halogénés et des esters organiques ou des mélanges de ces solvants.

**6.** Procédé selon la revendication 5, **caractérisé en ce que** le solvant est choisi parmi le n-pentane, le n-hexane, le n-heptane, le n-octane, le benzène, le toluène, le o-, le m-, le p-xylène, l'éthylbenzène, l'isopropylbenzène, le tert-butylbenzène, l'éther méthyléthylique, l'éther diéthylique, l'éther diisopropylique, l'éther di-n-butylique, l'éther méthyl-tert-butylique, l'éther cyclohexylméthylique, l'éther diphénylique, le furanne, le tétrahydrofuranne, 1,4-dioxanne, le tétrachlorométhane, le trichlorométhane, le dichlorométhane, le chloropentafluoroéthane, le 1,2-dichlorotétrafluoroéthane, l'hexafluoroéthane, le pentachloroéthane, le 1,1,2,2-tétrachloroéthane, le 1-bromo-2-chloroéthane, le 1,2-dichloroéthane, le 1,2-dichloropropane, le carbonate de diméthyle et le carbonate de diéthyle, ou des mélanges de ces solvants.

**7.** Procédé selon l'une quelconque des revendications 1 à 6, **caractérisé en ce que** le solvant est éliminé par évaporation sous pression normale ou sous pression réduite, par congélation, par lyophilisation, par filtration et par séchage ultérieur ou bien par séchage à température élevée sous pression normale ou de préférence sous pression réduite.

**8.** Utilisation de l'oxyde de zinc dont la surface a été modifiée, préparé conformément aux revendications 1 à 7, pour la préparation de dispersions stables d'oxyde de zinc dans des solvants organiques, qui présentent un moment dipolaire inférieur ou égal à 2,0 D.

**9.** Utilisation de dispersions d'oxyde de zinc selon la revendication 8, pour l'enduction de surfaces.

**10.** Utilisation de l'oxyde de zinc dont la surface a été modifiée, préparé conformément aux revendications 1 à 7, dans des agents cosmétiques pour l'entretien ou pour la protection de la peau, en particulier pour la protection contre l'effet du soleil.

**11.** Utilisation de l'oxyde de zinc dont la surface a été modifiée, préparé conformément aux revendications 1 à 7, à titre de substance active antimicrobienne.

**12.** Utilisation de l'oxyde de zinc dont la surface a été modifiée, préparé conformément aux revendications 1 à 7, dans des agents pharmaceutiques.

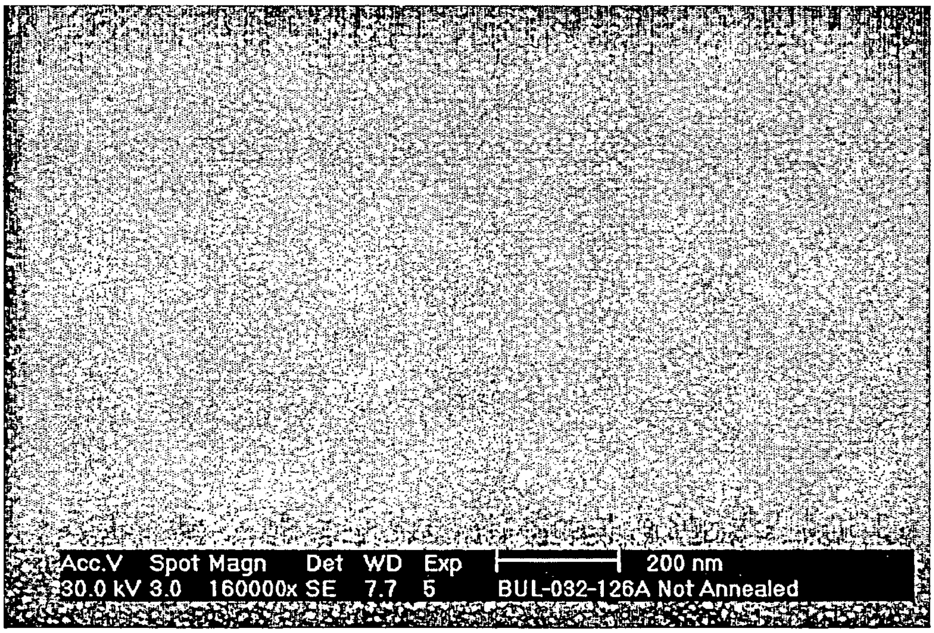


**Fig. 1:** REM-Aufnahme einer Zinkoxid-Schicht nach Auftragen auf eine Glasoberfläche. Der mittlere Teilchendurchmesser beträgt etwa 8 nm.

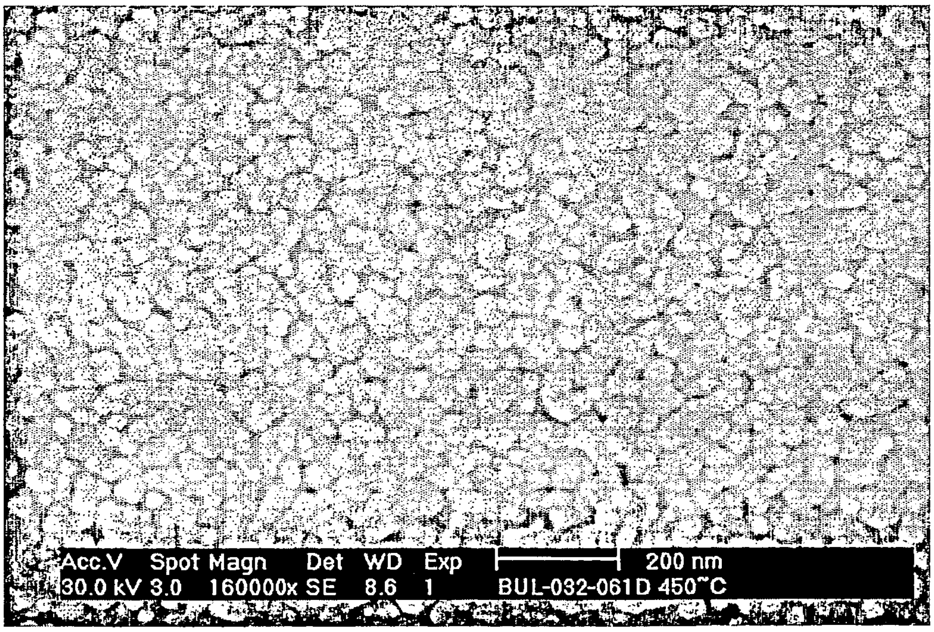


**Fig. 2:** REM-Aufnahme der Zinkoxid-Schicht aus Fig. 1 nach Tempern bei 450 °C für 1 Stunde. Der mittlere Teilchendurchmesser beträgt etwa 40 nm.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19907704
- WO 0249684 A
- WO 0014302 A
- DE 1165574 C


### In der Beschreibung aufgeführte Nicht-Patentliteratur


- **C. E. KRILL ; R. BIRRINGER.** Measuring average grain sizes in nanocrystalline materials. *Phil. Mag. A,* 1998, vol. 77, 621
- **L. BRUS.** *J. Phys., Chem.,* 1986, vol. 90, 2555-2560